# EUROPEAN PATENT SPECIFICATION

(11) **EP 1 712 194 B1**
(45) Date of publication and mention of the grant of the patent: **09.12.2009**
(21) Application number: 04708369.6
(22) Date of filing: 05.02.2004
(51) Int. Cl.: A61B 17/16

(54) **TOOLS FOR LOW-SPEED MILLING WITHOUT IRRIGATION AND WITH EXTRACTION AND RECOVERY OF TISSUE PARTICLES**
GERÄTE ZUR EXTRAKTION UND SAMMLUNG VON GEWEBEPARTIKELN BEI KÜHLUNGSLOSER BOHRMETHODE MIT NIEDRIGER DREHGESCHWINDIGKEIT
OUTILS DE FRAISAGE A BAS REGIME, SANS REFROIDISSEMENT LIQUIDE ET AVEC EXTRACTION ET RECUPERATION DE PARTICULES DE TISSU

(43) Date of publication of application: 18.10.2006
(73) Proprietor: BTI, I+D, S.L., 01005 Vitoria (ES)
(72) Inventor: ANITUA ALDECOA, Eduardo, E-01005 Vitoria (ES)
(74) Representative: Urteaga Simarro, José Antonio
(86) International application number: PCT/ES2004/000048
(87) International publication number: WO 2005/074816

(56) References cited:
- WO-A-2004/014241
- DE-A- 10 048 575
- DE-U- 8 415 145
- US-A- 5 429 504
- US-A- 6 068 632
- PATENT ABSTRACTS OF JAPAN vol. 1995, no. 02, 31 March 1995 (1995-03-31) & JP 6 304187 A (NIKON CORP), 1 November 1994 (1994-11-01)

## Description

The invention refers to techniques used in the drilling or milling of bone, cartilage, and other tissues for desirable medical purposes generally having to do with the implantation of prosthesis, osteosynthesis screws or other elements on knees, hips, the spine and other bones or tissues. One of the most important applications is the drilling of the maxillary bone of a patient in order to prepare it for a dental implant, in the fields of dental implantology and maxillofacial surgery.

When applied specifically in dental implantology the milling procedure involves the gradual drilling of the bone through the gradual insertion of mill bits of increasing diameters in order to form a cavity adapted to the dimensions of the implant or prosthesis. The milling procedure involves rotating the tool or mill bit at the required speed. The exact rotation speed parameter is determined by a number of factors, mainly the geometrical characteristics of the mill bit used and the sequence of the milling process phases.

Mill bits are available in a large number of shapes and sizes. This is mainly because each implant design usually comprises specific designs for specialised mill bits to mill cavities perfectly adapted to the dimensions of the implant.

It is widespread practice during the milling process to use mill bits with a range of different diameters for the same implant. This ensures that the mill bits used in each phase of the process adapt perfectly to the dimensions of the cavity to be drilled.

The wide variety of implants and mill bits used in conventional techniques means, therefore, that a broad range of milling speeds - from 800 to 1,500 rpm approximately - is used. This high-speed milling, as it is referred to, causes both the mill bit and the bone tissue it operates on to heat up with the temperature of the mill bit exceeding 40°C on occasions. Bone tissue cells are thermosensitive and have an optimal temperature of 37 °C. Any increase in this temperature can, therefore, be injurious to the cells and cause necrosis in many cases.

The thermal insult to which the tissue surrounding the implant is subjected during high-speed milling - to which the mechanical insult of the entire milling process must be added - has a damaging effect on the initial state of the cavity housing the implant. As a consequence, it takes longer for the bone to regenerate and bone-implant integration to occur, factors that impact on the level of success of the operation.

It is, therefore, widespread practice in conventional milling techniques to apply a saline irrigation solution on the mill bit and the drilling area in order to prevent the bit and the surrounding tissue from heating up. However, this irrigation solution washes away signalling proteins and other soluble substances that play an active part in bone regeneration.

These substances are released by the tissue in the area where tissue damage has occurred as a response to the insult and as a means of maintaining homeostasis, i.e. maintaining the biological and physico-chemical conditions prior to the insult, and are essential if the tissue is to recover. The specific physiological function of the signalling proteins is to transmit activation signals to the cell so that it can react to the deterioration suffered in the microenvironment. These proteins are connected to the extracellular matrix. This connection is broken when the mill bit impacts against the matrix. These signalling proteins are characterised by their low molecular weight and their solubility. A saline irrigation solution easily dissolves and washes them away, therefore, stripping the tissue of the natural resources it uses to heal itself.

Furthermore, it is common practice to extract and collect the particles of tissue removed during milling and use them in autografting. Such a technique renders unnecessary the use of alternative and far less useful techniques marketed for this purpose such as allografting (homologous grafting of tissue obtained from a human tissue bank) or xenografting (the process of grafting tissue from one species of animal to another). As part of this process a suction device equipped with a filter collects all the bone particles. Following repeated analysis of high-speed milling procedures it was found that the cells in these particles had died off as a result of the thermal and mechanical insults suffered during the process.

The main objective of this invention is to provide a tool for a milling procedure that protects the tissue surrounding the drilling area as much as possible, prevents the area from heating up and, at the same time, negates the secondary effects deriving from the use of saline irrigation solution - mainly the washing away of the intrinsic cellular signals that help the tissue heal more quickly and become biologically stronger.

Another objective of this invention is to provide a tool for a milling procedure that allows the particles of tissue removed during drilling to be collected and then used to prepare an effective autografting process. As a result, this invention aims to define and use tools that are designed to retain and not expel particles.

Another objective of this invention is to define a tool for a milling procedure that constantly adapts itself to the characteristics of the specific area of tissue being drilled. This objective, which all milling procedures must comply with, is based on the fact that the outer surface of the tissue (the cortical layer, where milling begins), is harder and contains fewer cells. Once this layer has been drilled the tissue becomes less dense and contains more cells. If this objective is reached, it will be easier to make the implant stable in an initial phase thereby aiding tissue-implant integration.

The invention also aims to design milling tools that are optimum for performing the milling procedure of the present invention.

In order to reach the aforementioned objectives, it is defined a procedure for milling the tissue of a patient with a view to creating a cavity designed to house an implant or prosthesis. This procedure involves low-speed, non-irrigation milling. As a result of the process, tissue particles with a high biological quality are obtained and subsequently used as autografts after being mixed preferably with PRGF (Plasma Rich in Growth Factors), obtained in accordance with invention WO0044314 awarded to this applicant.

In order to form the cavity housing the implant the milling procedure consists, in the main, of three milling phases:
1) Initial phase
2) Intermediate phase
3) Countersinking phase

The initial phase involves cutting through the cortical tissue - the first layer of tissue and generally characterised as being very hard in consistency. This initial phase already forms part of other procedures. To ensure correct implementation of this phase a conical 'starter mill bit' must be used. This bit is specially designed to penetrate cortical tissue and to make it easier to start milling cavities even in small areas of tissue. The tip of the mill bit must be very sharp, therefore, to enable the drill hole to be made in exactly the right position. An appropriate mill bit to be used in this phase is that of international application PCT/ES03/00443, also in favour of the present applicant.

Initial-phase milling is usually carried out at a high speed, preferably ranging between 800 and 1,200 rpm. This is due to the design of the mill bit, which has a very sharp point, and to the fact that the mill bit must make a very fine drill hole in hard tissue without sliding around. Copious amounts of a physiological serum irrigation solution need to be applied in this initial phase in order to prevent the tissue heating up as a result of the high-speed milling.

The aim of the intermediate phase is to form practically the entire cavity housing the implant, with its depth, width and other key aspects being defined in the process. As explained in the introduction to this invention, mill bits of various shapes and sizes are needed to mill a cavity that is exactly the same size and shape as the implant. As a result, several types of mill bits are used in the intermediate phase until the required cavity is formed.

The intermediate milling phase is characterised by two main factors:
- Milling is carried out at low speeds of between 20 and 80 rpm and without a saline irrigation solution being applied.
- The tissue displaced or released during this intermediate cavity-defining phase is extracted or collected. The invention achieves this through the use of specially designed mill bits that allow the displaced tissue to be retained in the mill bit during milling, thus making it easier to extract. The following method is frequently used: milling is momentarily interrupted when the device detects that the mill bit contains a sufficient amount of tissue particles or whenever a pause is considered necessary. The mill bit is then removed from the cavity and a spatula or other instrument is used to extract the tissue from the bit before it is deposited in a small container made of glass or a similar sterile material. Milling work then resumes. This means that the tissue particles can be collected without having to use suction filters or other additional tools.

In the countersinking phase a base to receive the implant or prosthesis is created if necessary. The function of the countersinking phase is to open out the top of the cavity to create enough space to house the head of the implant when it is fitted into the cavity. The shape of the implant determines the point at which the countersinking phase - if deemed necessary - takes place. In some cases, countersinking occurs at the end of the intermediate phase or when the cavity is being defined whereas in other cases it takes place during the intermediate phase.

As in the intermediate phase, milling during the countersinking phase is carried out at low speeds of between 20 and 80 rpm and without a saline irrigation solution being applied. Additionally, the tissue displaced or released during this countersinking phase is extracted or collected through the use of specially designed mill bits that allow the displaced tissue to be retained in the mill bit during milling, thus making it easier to extract. The design of these mill bits used during the countersinking phase has the same characteristics as the design of the tools used in the intermediate milling phase.

Although initial-phase milling should preferably be carried out at high speeds, low-speed milling - as in the intermediate and countersinking phases - is also acceptable if required by the specific application or case.

As regards the tools or mill bits used in the procedure and as mentioned previously, the invention defines specific mill bits for intermediate- and countersinking-phase milling. These mill bits have a retentive design that enables the storage and subsequent retrieval of displaced or extracted tissue.

The mill bits used in the intermediate and countersinking phases are all predominantly slender, cylindrical parts featuring, at the top, a smooth area of standard dimensions to connect the bit to a rotating motor. Secondly, the bit features a milling section consisting of spiral grooves which have been cut to create the angle required to release or extract the cut material and, at the same time, to create spaces to house the extracted tissue. Thirdly, the mill bit features of a sharp tip or apex in case of intermediate-phase mill bits, and a non-sharp tip or apex in case of countersinking-phase mill bits.

The spaces housing the extracted tissue - or tissue retention areas - are formed between the continuous loops of the spiral grooves, on the internal concave surface in-between these loops. The following features of this section of the mill bit account for its retentiveness or the ability of these areas to store extracted tissue:
- Firstly, the spiral grooves are cut at an angle of inclination of between 25 and 40 degrees in relation to the longitudinal axis of the mill bit, in contrast to conventional mill bits where the spiral is usually inclined at an angle of 6 degrees and sometimes up to 15 degrees.
- Secondly, the concavity of the retention areas towards the core of the mill bit or the longitudinal axis is more pronounced than in conventional, non-retentive mill bits such as that of JP6304187, whose retention areas seen in a cross-sectional view are only a portion of a circle, more precisely around 15-20% of a circle (30-40% of a semicircle). More specifically, in a cross-section of this area of the mill bit, the curvature or concavity of the retention areas is approximately the same shape as a semi circumference and can even be greater or more closed.

The definition and use of mill bits with these characteristics ensures that the tissue extracted during the milling procedure is directed towards the retention areas and, as no irrigation solution is applied, is housed in them.

Nevertheless, it should be pointed out that in this invention the retentive nature of the mill bit and the fact that milling is carried out at low speeds and without irrigation makes it easier to obtain tissue particles that can be used for autografting. The fact that milling is performed at low speeds helps improve, therefore, the quality of tissue obtained, as the particles are larger and contain a significantly larger number of living cells than particles obtained as a result of high-speed milling. This is due to the fact that as the mill bit completes considerably more rotations at high speed than at low speed yet advances the same distance, the tissue is shredded to a greater extent, thus forming a type of powder which, as test have shown, contains no living cells. Furthermore, the fact that a saline irrigation solution is not applied in order to cool the mill bit and the surrounding area means that the signalling proteins and other substances accelerating and encouraging tissue regeneration and enabling the rapid stabilisation of the implant are not eliminated from the surrounding tissue.

In addition, all the mill bits, apart from the starter mill bit - which has a shorter milling section - may feature strips cut or etched into the outer surface that indicate the different milling depths in accordance with the height of the implants. As the mill bit rotates at low speeds, these strips remain visible and can, therefore, be used to indicate the point at which the corresponding depth for each particular mill bit has been reached and, therefore, the point at which milling should stop or continue using the next mill bit.

The low-speed, non-irrigation milling procedure and tools according to the invention used to extract and collect tissue particles, not only meet the stated objectives but also provide other proven advantages and positive features which are detailed below.

Tests have shown that the milling technique does not cause the temperature of the mill bits to increase by more than five degrees centigrade, which, when added to the ambient temperature, means that the temperature is kept below 40 degrees centigrade - the point at which insult and even necrosis occurs.

Tests using optical and electronic microscopes have also shown that the bone particles extracted during the milling process retain their osteogenic (deriving from bone-forming tissue), osteoinductive (inducing other cells to form bone) and osteoconductive (providing structural support during bone regeneration) properties. The bone particles are, therefore, ideally suited for use in autografting. Autografting, for example, can be performed by mixing the bone particles with PRGF (Plasma Rich in Growth Factors, according to invention WO0044314 awarded to this applicant). Another potential autografting method involves keeping the particles in physiological serum or in the patient's blood. The mixture can later be used in autografting.

The low-speed, non-irrigation milling procedure can be applied not only in implantology but also in orthopaedic surgery and traumatology, specialised fields in which highly aggressive surgical approaches are traditionally used based on high-speed milling and mechanical criteria that fail to take the biological insult caused to the tissue into account.

In this respect, technical innovations aimed at reducing the insult can contribute greatly to improved clinical development and a shorter recovery period (as has already been proven with the application of the technique detailed in patent WO0044314, awarded to this applicant, to this type of operation). They also enable a large quantity of living bone to be obtained for use in autografting. In the case of hip and knee prostheses, the use of a low-speed, non-irrigation milling procedure allows a large quantity of bone to be recovered for use as a prosthesis graft. The graft can be applied using rods or pins, osteosynthesis screws or microplates in the event of bone fractures.

Details of the invention can be seen in the diagrams attached although said diagrams do not show all the contents of the invention:
- Figure 1 shows an initial example of a low-speed milling procedure.
- Figure 2 shows a second example of a low-speed milling procedure.
- Figure 3 shows the possible arrangement of a milling tool according to the invention.
- Figure 4 shows the possible arrangement of another milling tool.

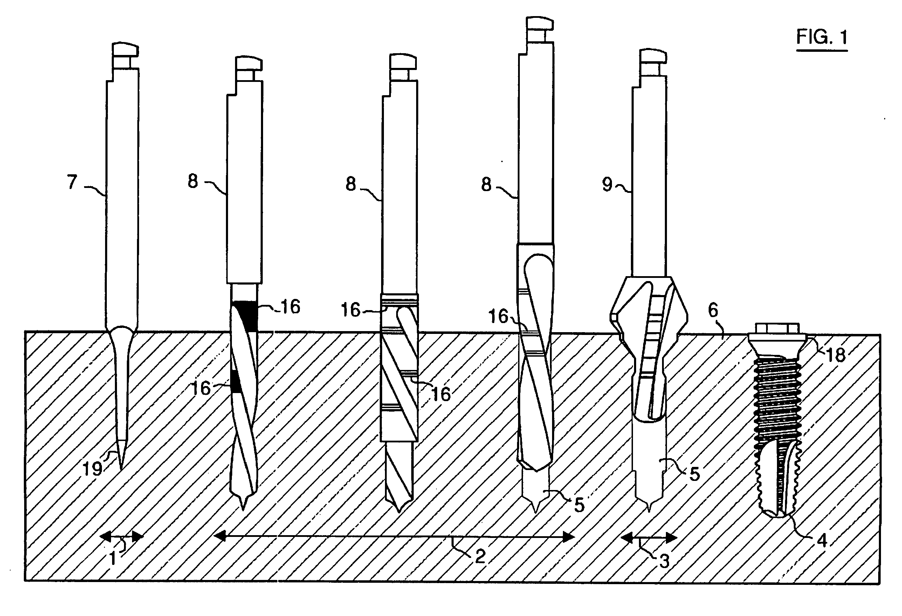

Figure 1 shows an example of a low-speed milling procedure in which the procedure is applied in order to form a cavity or alveolus (5) in the tissue (6) of the patient. In this particular case the tissue is the maxillary bone and the cavity is formed in order to house a dental implant (4). In this procedure, the cortical layer or the hardest outer section of the bone (6) is drilled as part of an initial phase (1). This is followed by an intermediate milling phase (2) in which the cavity (5) is defined. Finally, the procedure is brought to a close by a countersinking phase (3) in which the cavity (5) is widened in order to house the head (18) of the dental implant (4).

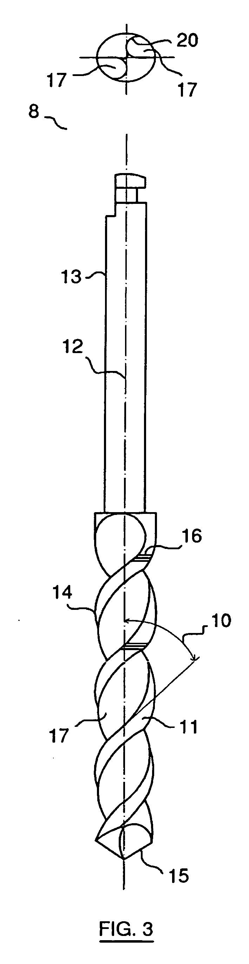

As has been pointed out in this description of the invention, the requisite tools are used in each milling phase in order to produce the desired effect on the cavity, etc. In this respect, the starter mill bit (7) used during the initial phase (1) features a very sharp, conical tip (19) enabling it to start milling the cavity. Furthermore, the countersinking mill bit (9) used during the countersinking phase (3) is shorter and wider than the other mill bits as its function is to open out the top of the cavity (5). The shape of the intermediate mill bits (8) used during the intermediate phase (2) are extensively detailed in figure 3. Figure 1 shows the layout of marks (16) on the surface of the mill bit (8) that indicate the depth to which each tool should drill or mill. These marks act as a guide for the specialist carrying out the milling.

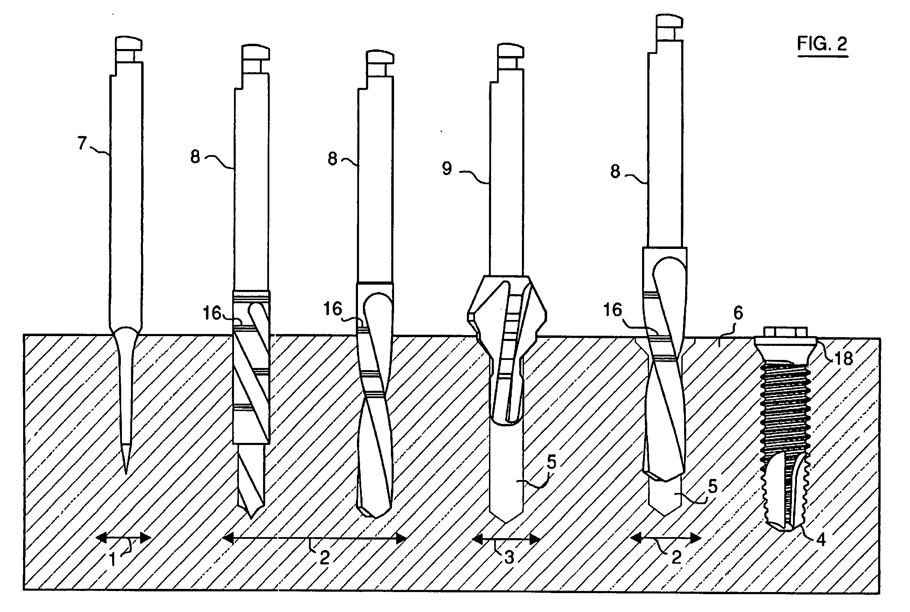

Figure 2 details another example of the procedure, for the same application as shown in figure 1, which sets out to show how the countersinking phase (3) can be implemented during the intermediate phase (2), an option that may be useful for certain types of dental implants (4), depending on the types of tools available.

Figure 3 provides an example of an intermediate mill bit (8) used during the intermediate phase of the procedure and during which most of the cavity in the patient's tissue is formed. This intermediate mill bit (8) consists mainly of three parts or zones: The top of the mill bit features a smooth, predominantly cylindrical area (13) of standard dimensions. Secondly there is the mill section (14) featuring spiral grooves (11) cut into the mill. Thirdly, the mill features a sharp tip or apex (15).

The mill bit according to the invention features retention areas (17) that correspond with the interior of the spiral grooves (11) towards which the tissue extracted during the milling process moves before finally being housed inside them. The retentiveness of these retention areas (17) is enhanced by the fact that the spiral grooves (11) in the intermediate mill bit (8) are formed in such a way that the angle (10) at which they are inclined in relation to the longitudinal axis (12) of the mill bit is between 25 and 40 degrees, and by the fact that the cross-section of the curvature (20) of the retention areas is more closed than a semi circumference.

As can be seen in the figure, the mill bit (8) also features marks (16) that indicate the depth to which each tool should drill or mill, thus acting as a guide for the specialist carrying out the milling.

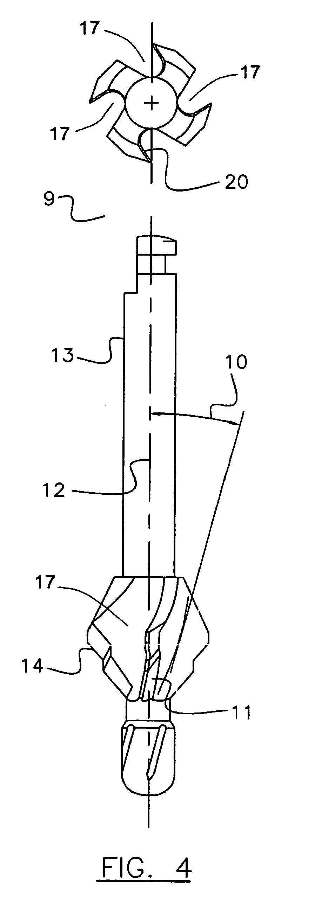

Figure 4 provides an exemplary embodiment of a mill bit (9) to be used during the countersinking phase of the procedure due to its wider mill section (14), which provides the definition of a wider opening at the top of the cavity, thus creating a space to house the head of the implant. This countersinking-phase mill bit (9) consists mainly of two parts or zones: a smooth, predominantly cylindrical area (13) of standard dimensions, and the mill section (14) featuring spiral grooves (11) cut into the mill.

The countersinking-phase mill bit (9) features retention areas (17) that correspond with the interior of the spiral grooves (11) to which the tissue extracted during the milling process moves is attracted and finally housed in. The retentiveness of these retention areas (17) is enhanced by the fact that the spiral grooves (11) in the countersinking-phase mill bit (9) are formed in such a way that the angle (10) at which they are inclined in relation to the longitudinal axis (12) of the mill bit is between 25 and 40 degrees, and by the fact that the cross-section of the curvature (20) of the retention areas is approximately the same shape as a semi circumference. According to the invention, it is more closed.

## Claims

1. Milling tools (8, 9) to be used in a milling procedure to be performed on bone, cartilage or other tissue (6) in order to form a cavity (5) of a shape and size that allows it to house an implant (4) or for other purposes in which a cavity (5) needs to be formed, with said milling tools (8, 9) being predominantly longitudinal tools that comprise an area (14) featuring spiral grooves (11), wherein tissue retention zones (17) are formed between the spiral grooves (11) in order to store the tissue extracted during the milling process, wherein the retention zones (17) are concave or curved inwards, **characterized in that**, when seen in a cross-section, the retention zones (17) are more closed than a semi circumference.

2. Milling tools (8, 9) according to claim 1 **characterised in that** the spiral of the spiral grooves (11) is formed at an angle of inclination (10) of between 25 and 40 degrees in relation to the longitudinal axis (12) of the milling tool (8, 9).

3. Milling tools (8, 9) according to claim 1 **characterised in that** the tools (8, 9) feature one or more visible horizontal marks (16), formed in relief or in another appropriate form, to serve as a guidance during the milling process.

## Patentansprüche

1. Fräswerkzeuge (8, 9) zur Verwendung bei einem Fräsverfahren, das an Knochen, Knorpel oder anderem Gewebe (6) durchgeführt wird, die der Bildung eines Hohlraums (5) von einer Form und Größe dienen, welche die Unterbringung eines Implantats (4) gestattet, oder für andere Zwecke, bei denen ein Hohlraum (5) gebildet werden muss, wobei besagte Fräswerkzeuge (8, 9) vorherrschend longitudinale Werkzeuge sind, die eine Fläche (14) mit spiralförmigen Kerben (11) umfassen, wo Bereiche für die Gewebeerhaltung (17) zwischen den spiralförmigen Kerben (11) gebildet werden, um das während des Fräsverfahrens extrahierte Gewebe zu lagern, wobei die Bereiche für die Gewebeerhaltung (17) nach innen ausgehöhlt oder gekrümmt sind, so dass diese Bereiche für die Erhaltung (17) - im Querschnitt betrachtet - geschlossener sind als ein Halbkreisumfang.

2. Fräswerkzeuge (8, 9) in Übereinstimmung mit Anspruch 1, **dadurch gekennzeichnet, dass** die Spirale der spiralförmigen Kerben (11) in einem Neigungswinkel (10) von 25 bis 40 Grad in Bezug auf die Längsachse (12) des Fräswerkzeugs (8, 9) geformt sind.

3. Fräswerkzeuge (8, 9) in Übereinstimmung mit Anspruch 1, **dadurch gekennzeichnet, dass** die Werkzeuge (8, 9) einen oder mehrere sichtbare horizontale Markierungen (16) aufweisen, die als Relief oder in einer anderen geeigneten Form ausgebildet sind, und die als Führung während des Fräsverfahrens dienen sollen.

## Revendications

1. Outillages de fraisage (8,9) à utiliser dans le cadre d'une opération de fraisage à effectuer sur un os, sur du cartilage ou sur tout autre tissu (6) ont pour objet de creuser une cavité (5) de forme et de taille permettant de loger un implant (4), ou pour tout autre fonction où il est nécessaire de creuser une cavité (5), ces outillages de fraisage (8,9) étant essentiellement des outils longitudinaux comprenant une extrémité (14) avec des rainures hélicoïdales (11), où zones de rétention des tissus (17) se forment entre les rainures hélicoïdales (11) pour emmagasiner les tissus extraits lors du processus de fraisage, où les zones de rétention (17) sont concaves ou incurvées à l'intérieur, **caractérisés par le fait que**, telles qu'elles figurent sur la coupe transversale, les zones de rétention (17) sont plus refermées qu'un demi-cercle.

2. Outillages de fraisage (8,9), conformément à la revendication 1, **caractérisés par le fait que** la spirale des rainures hélicoïdales (11) est formée en angle d'inclinaison (10) entre 25 et 40 degrés par rapport à l'axe longitudinal (12) des outillages de fraisage (8,9).

3. Outillages de fraisage (8,9), conformément à la revendication 1, **caractérisés par le fait que** sur les outillages (8,9) figurent une ou plus de stries horizontales visibles (16), en relief ou sous toute autre forme appropriée, servant de guidage lors du processus de fraisage.
